# EUROPEAN PATENT SPECIFICATION

(11) **EP 4 014 870 B1**
(45) Date of publication and mention of the grant of the patent: **03.07.2024**
(21) Application number: 21215007.2
(22) Date of filing: 16.12.2021
(51) Int. Cl.: A61B 5/367, A61B 5/287, A61B 5/339, A61B 18/12

(54) **GUI FOR HIGHLIGHTING SIGNALS PRIOR, DURING, AND POST ABLATION USING MULTI-ELECTRODE CATHETERS**
GRAFISCHE BENUTZEROBERFLÄCHE ZUM HERVORHEBEN VON SIGNALEN VOR, WÄHREND UND NACH DER ABLATION MITTELS MEHRELEKTRODENKATHETERN
IUG POUR LA MISE EN ÉVIDENCE DES SIGNAUX AVANT, PENDANT ET APRÈS UNE ABLATION À L'AIDE DE CATHÉTERS MULTI-ÉLECTRODES

(30) Priority: 17.12.2020 US 202017124679
(43) Date of publication of application: 22.06.2022
(73) Proprietor: Biosense Webster (Israel) Ltd., Yokneam, 2066717 (IL)
(72) Inventor: GOVARI, Assaf, 2066717 Yokneam (IL); ALTMANN, Andres Claudio, 2066717 Yokneam (IL); GOLDBERG, Aviva, 2066717 Yokneam (IL)
(74) Representative: Carpmaels & Ransford LLP

(56) References cited:
- US-A1- 2015 141 978
- US-A1- 2017 065 339
- US-A1- 2017 120 048
- US-A1- 2018 310 987
- US-A1- 2018 368 927
- US-A1- 2019 307 323

## Description

### FIELD OF THE INVENTION

The present invention relates generally to presenting data from medical probes in real-time, and particularly to presenting in real-time signals from multi-electrode Electrophysiological (EP) sensing and ablation catheters using a graphical user-interface (GUI).

### BACKGROUND OF THE INVENTION

Presenting data related to a specific electrode of an invasive probe was previously proposed in the patent literature. For example, U.S. Patent Application Publication 2015/0320478 describes high-frequency ablation of tissue in the body using a cooled electrode. The ablation system includes a computer graphic control system adapted to display on a real time graphic display a measured parameter related to the ablation process and visually monitor the variation of the parameter of ablative signal output during the ablation process. In one example, one or more measured parameters of multiple return electrodes are displayed simultaneously to visually interpret the relation of their variation and values. The displayed one or more parameters can be taken from the list of measured voltage, current, power, impedance, electrode temperature, and tissue temperature related to the ablation process. The graphic display gives the clinician an instantaneous and intuitive feeling for the dynamics and stability of the ablation process for safety and control.

As another example, U.S. Patent Application Publication 2017/0065339 describes catheter systems and methods for the selective and rapid application of DC
voltage to drive irreversible electroporation. In some embodiments, an apparatus includes a medical device including a series of electrodes. A selection module of the apparatus is configured identify at least one electrode as an anode and at least one electrode as a cathode. In an embodiment, when the device is applying pulses, a trigger button flashes in sequence with the pulse delivery in a specific color. The waveform of each delivered pulse is displayed on the touchscreen interface. A graphic representation of the pre and post impedance between electrodes involved in the sequence can also be shown on the interface.

U.S. Patent Application Publication 2016/0278660 describes methods and devices for identifying a treatment site. The methods may include engaging a plurality of electrodes with plurality of locations on or adjacent an interior wall of a patient. The methods may also include generating a pacing stimulus through a first pair of the plurality of electrodes and measuring a resulting electrical activity. The methods may also include identifying at least one site for treatment based on the resulting electrical activity. In some examples, a determination of electrodes for further use may be made by an operator reviewing a representation of an electrical activity displayed on interface.

US 2018/368927 A1 describes a computer-implemented method for generating and displaying a graphical user interface (GUI). The method includes displaying, via the GUI, a real-time video received from a camera disposed within an ablation catheter. The method further includes displaying, via the GUI, a graphical representation of a plurality of electrodes of the ablation catheter.

US2015141978 describes ablation devices and methods for determining a degree of contact between an electrode and a target tissue. An example ablation device for treating body tissue may include a catheter having a proximal end region and a distal end region. An electrode may be disposed adjacent to the distal end region of the catheter. The device may also include a processing unit having a memory. The processing unit may be in electrical communication with the electrode. The processing unit may be capable of determining a degree of contact between the electrode and a target tissue.

### SUMMARY OF THE INVENTION

An embodiment of the present invention that is described hereinafter provides a system including a display and a processor. The processor is configured to (a) receive multiple signals sensed by multiple electrodes of a multi-electrode catheter in a heart of a patient, (b) determine which of the electrodes are to be active in an ablation
procedure, and (c) present the multiple signals to a user, on the display, using a graphical user interface (GUI) that (i) visualizes the signals from the electrodes determined to be active by a first graphical feature, and (ii) visualizes the signals from the electrodes determined not to be active by a second graphical feature, different from the first graphical feature.

In some embodiments, the processor is configured to visualize a signal using the first graphical feature by plotting the signal with a first line style, and using the second graphical feature by plotting the signal with a second line style, different from the first line style. In other embodiments, the processor is configured to visualize the signals from the electrodes determined not to be active by partially blanking the signals.

In an embodiment, the processor is further configured to automatically update the visualization of the signals from the electrodes based on determining level of physical contact of electrode with tissue.

In another embodiment, the processor is further configured to indicate to the user, using the GUI, which of the electrodes are determined to be active.

In some embodiments, the processor is configured to indicate which of the electrodes are determined to be active on a graphical illustration of the catheter.

In some embodiments, wherein the signals are electrograms.

There is additionally provided a method (not explicitly claimed as such though nevertheless useful for understanding the invention) including receiving multiple signals sensed by multiple electrodes of a multi-electrode catheter in a heart of a patient. A determination is made as to which of the electrodes are to be active in an ablation procedure. The multiple signals are presented to a user on a display, using a graphical user interface (GUI), by (i) visualizing the signals from the electrodes determined to be active by a first graphical feature, and (ii) visualizing the signals from the electrodes determined not to be active by a second graphical feature, different from the first graphical feature.

There is further provided, in accordance with another embodiment of the present invention, a computer software product, the product including a tangible non-transitory computer-readable medium in which program instructions are stored, which instructions, when executed by a processor, cause the processor to (a) receive multiple signals sensed by multiple electrodes of a multi-electrode catheter in a heart of a patient, (b) determine which of the electrodes are to be active in an ablation procedure, (c) present the multiple signals to a user on a display, using a graphical user interface (GUI) that (i) visualizes the signals from the electrodes determined to be active by a first graphical feature, and (ii) visualizes the signals from the electrodes determined not to be active by a second graphical feature, different from the first graphical feature.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a multi-electrode catheter-based position-tracking and ablation system comprising a GUI configured to blank signals of electrodes that are not selected for ablation, in accordance with an exemplary embodiment of the present invention;
Figs. 2A and 2B are schematic, pictorial illustrations of the GUI of Fig. 1, in two selected electrode configurations, in accordance with some embodiments of the invention; and
Fig. 3 is a flow chart that schematically illustrates a method for using the GUI of Fig. 2.

### DETAILED DESCRIPTION OF EMBODIMENTS

### OVERVIEW

A multi-electrode catheter may be employed in various clinical applications, such as electro-anatomical mapping and ablation of the cavity walls. Ablation, such as irreversible electroporation (IRE), is typically of very short duration, and in many cases not all the electrodes of the multi-electrode catheter are activated. As a multi-electrode catheter may include tens of electrodes or more, and since each electrode is associated with a respective signal to monitor, it is difficult for an operator to see which signals (e.g., which subset of a set of electrograms acquired by the electrodes) should be observed for the ablation.

For example, the physician may receive visual indications for each electrode regarding sufficient or insufficient physical contact with cardiac tissue, indicating whether the electrode should be operated for ablation. As another example, in case of pulmonary vein isolation, the physician may receive an indication for some of the electrodes that, if operated for ablation, may cause collateral damage to nearby sensitive tissue, making monitoring signals from these electrodes an unnecessary overhead. In view of the above complicated scenarios that involve potential hazards, a physician may benefit from a visual input that identifies the electrodes selected for ablation, and highlights the signals (e.g., electrophysiological (EP) signals from contacted tissue) they sense.

Embodiments of the present invention that are described hereinafter provide the operator (e.g., a physician) with a graphical user interface (GUI) that selectively visualizes multiple signals sensed by multiple electrodes of a multi-electrode catheter, according to determined status of the electrodes.

In an embodiment, the GUI visualizes the signals from the electrodes determined to be active (e.g., suitable for use for ablation) by a first graphical feature. The GUI visualizes the signals from the electrodes determined not to be active (e.g., immersed in blood, and hence not suitable for use for ablation) by a second graphical feature, different from the first graphical feature. For example, the GUI highlights signals of electrode/channel determined to be active by being selected for ablation, and partially blanks the signals of electrodes determined to be inactive by not being selected for ablation. Both highlighting and blanking are available prior, during, and post ablation.

In some embodiments, the disclosed GUI illustrates the expandable frame (e.g., of a balloon) including an illustration of the multiple electrodes and their listing in numerical sequence, e.g., #1, #2, ... #N-1, #N. The GUI further provides a signal selection mode in which clicking on any given signal, or marking a group of signals (using a mouse or touch display or other suitable input device), blanks signals of electrodes not selected for ablation.

Typically, the processor is programmed in software containing a particular algorithm that enables the processor to conduct each of the processor-related steps and functions outlined above.

By providing the disclosed GUI, physician awareness of the ablation configuration selected is increased and thereby provides safer multi-electrode ablation treatments.

### SYSTEM DESCRIPTION

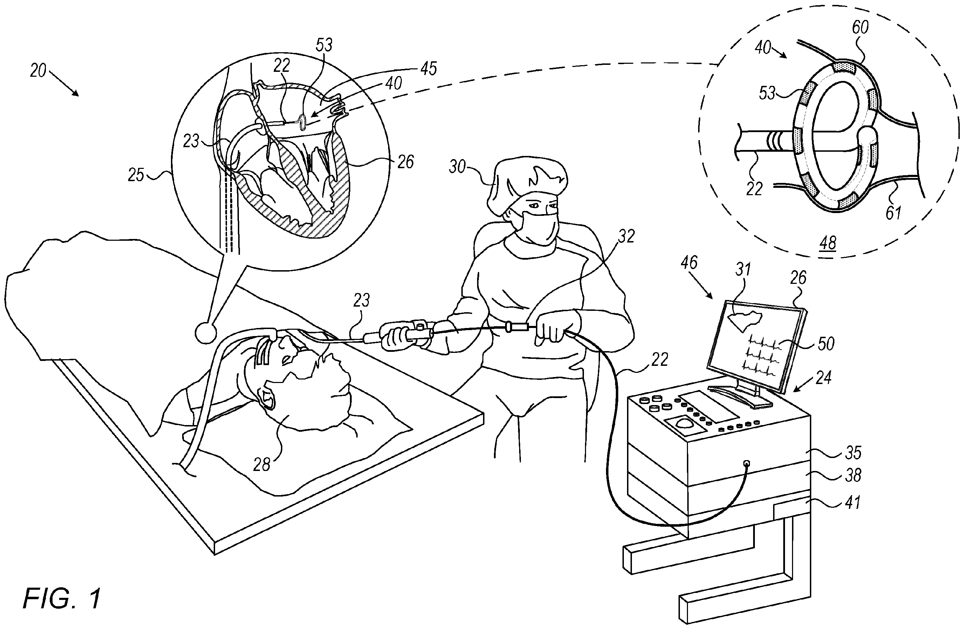

Fig. 1 is a schematic, pictorial illustration of a multi-electrode catheter-based position-tracking and ablation system 20 comprising a GUI 46 configured to blank signals of electrodes 53 that are not selected for ablation, in accordance with an embodiment of the present invention.

As seen, system 20 comprises a multi-electrode lasso catheter 40 that is fitted at a distal end of a shaft 22 of the catheter with lasso catheter 40 comprising multiple electrodes 53 (seen in insets 25 and 48). In the embodiment described herein, electrodes 53 are used by a physician 30 to perform at least one of the following procedures: (i) an electro-anatomical mapping of a cardiac chamber, such as a left atrium 45 of heart 26, and (ii) IRE ablation of tissue of an ostium 60 of a pulmonary vein 61 of a patient 28.

Physician 30 inserts shaft 22 through a sheath 23 into heart 26 of patient 28, and advances the distal end of shaft 22 to a target location in heart 26 by manipulating shaft 22 using a manipulator 32 near the proximal end of the catheter and/or deflection from the sheath 23.

During the procedure, a tracking system is used to track the respective locations of electrodes 53, such that each of the diagnostic signals may be associated with the location at which the diagnostic signal was acquired. A suitable tracking system is, for example, the Advanced Catheter Location (ACL) system, made by Biosense-Webster (Irvine, California), which is described in US Patent 8,456,182. In the ACL system, a processor estimates the respective locations of electrodes 53 based on impedances measured between each of the electrodes and a plurality of surface-electrodes that are coupled to the skin of patient 28.

Once the distal end of shaft 22 has reached target location (e.g., left atrium 45), physician 30 retracts sheath 23 and catheter 40 self-expands into its pre-formed arcuate shape. Physician 30 further manipulates shaft 22 to place electrodes 53 in contact with ostium 60 tissue.

During a mapping procedure, electrodes 53 acquire and/or inject signals from and/or to the tissue. A processor 38 in console 24 receives these signals via an electrical interface 35, and uses information contained in these signals to construct an electro-anatomical map 31 and ECG traces 50. During and/or following the procedure, processor 38 displays electro-anatomical map 31 and ECG traces 50 on a display 26. Typically, processor 38 stores electro-anatomical map 31 and ECG traces 50 in memory 41.

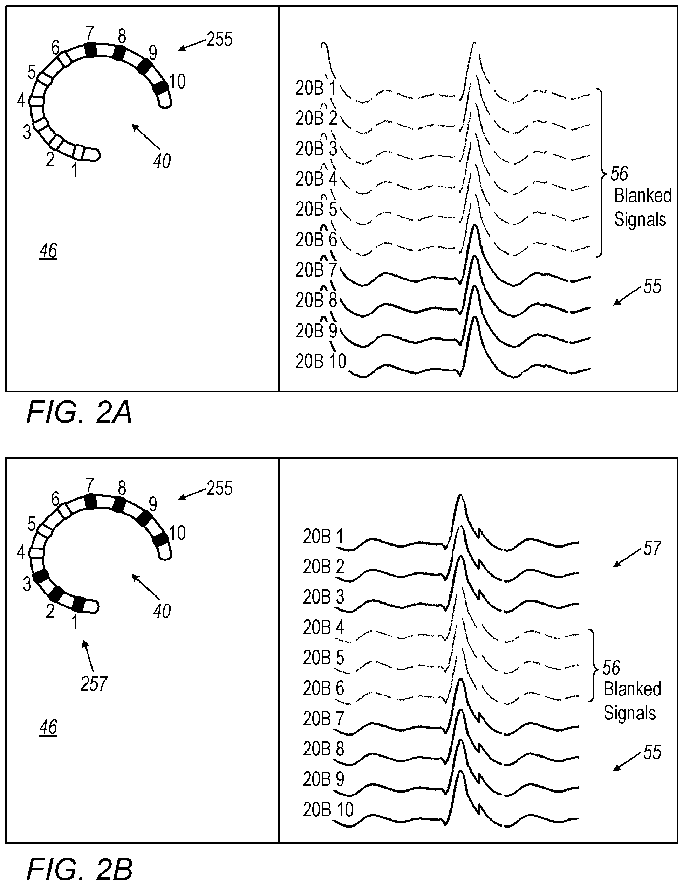

As further seen in inset 48, some of electrodes 53 of lasso catheter 40 are in contact with ostium 60 tissue while others are not (i.e., are in a blood pool) . In GUI 46, electrodes 53 that are in contact are shown highlighted, and physician 30 can blank ECG signals of electrodes 53 that are not in contact, as shown in Fig. 2.

Processor 38 presents GUI 46 which physician 30 can operate, for example, from a touch display 26, to see an illustration of catheter 40 and electrodes 53, and, using GUI 46 tools, enable or disable one or more electrodes 53 as ablation electrodes.

The example illustration shown in Fig. 1 is chosen purely for the sake of conceptual clarity. Other multi-electrode catheters may be used, such as balloon catheter, multi-arm catheter, and basket catheter.

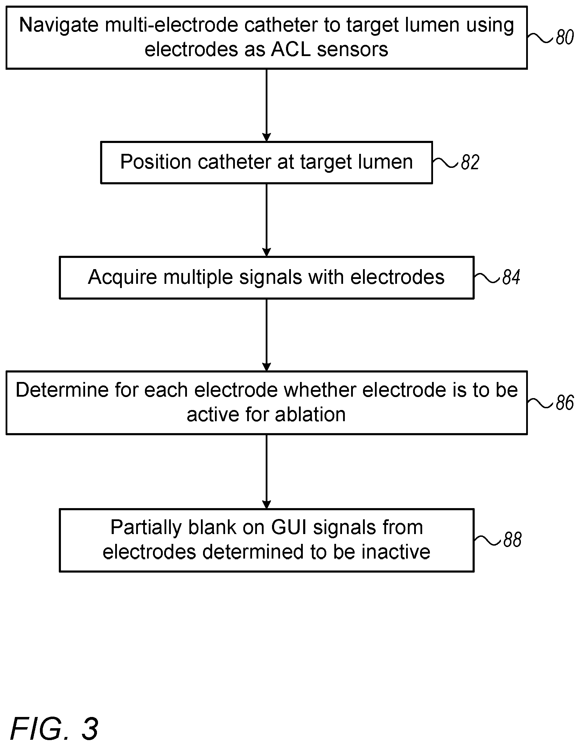

Processor 38 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 38 runs a dedicated algorithm as disclosed herein, included in Fig. 3, that enables processor 38 to perform the disclosed steps, as further described below.

### GUI FOR HIGHLIGHTING SIGNALS PRIOR, DURING, AND POST ABLATION USING MULTI-ELECTRODE CATHETERS

Figs. 2A and 2B are schematic, pictorial illustrations of GUI 46 of Fig. 1, in two selected electrodes 53 configurations, in accordance with some embodiments of the invention. In each of the figures, GUI 46 illustrates lasso catheter 40 with electrodes 53 including an electrode listing in numerical sequence, #1, #2, ... #9, #10. In Figure 2A, the electrodes selected for ablation are a group 255 of electrodes #7, #8, #9, #10, while in Fig. 2B the active electrodes (e.g., selected for ablation) are group 257 of electrodes #1, #2, #3, and group 255 of electrodes #7, #8, #9, #10.

As seen, electrogram signals 55 and 57 are highlighted, and electrogram signals 56 are partially blanked, according to the selected electrodes.

Figs. 2A and 2B give an example visualization. In this example the electrodes selected for ablation are shown as filled area electrodes, and the electrodes not selected for ablation are shown as empty area electrodes. Alternatively, however, any other GUI having any other suitable different graphical features can be used to distinguish between electrodes determined active for ablation and such determined inactive.

In an example embodiment, signals of active electrodes can be plotted with a first line style (e.g., full thick line), while signals of inactivate electrodes can be plotted with a second line style, different from the first line style (e.g., thin dashed line). As another example, signals from active electrodes can be presented with different color form the color used for signals of inactive electrodes.

Moreover, while in the embodiment shown in Figs. 2A and 2B the catheter is shown with the electrodes, in other embodiments only the signals are shown, with the distinctive visualization to indicate the active and inactive electrodes.

Furthermore, any other graphical means may be used, (e.g., relief icons of different shapes) to achieve the distinctive visualization.

Fig. 3 is a flow chart that schematically illustrates a method for using GUI 46 of Fig. 2. The method is not explicitly claimed as such, but is nevertheless useful for understanding the present invention. The algorithm, according to the presented method, carries out a process that begins when physician 30 navigates the multi-electrode catheter 40 to a target location within a lumen of a patient, such as at ostium 60, using, for example, electrodes 53 as ACL-sensing electrodes, at a balloon catheter navigation step 80.

Next, physician 30 positions catheter 40 at ostium 60, at a catheter positioning step 82. Next, at signal acquisition step 84, physician 30 acquires, using electrodes 53, multiple signals, such as EP signals and respective signals (e.g., impedances) indicative of level of physical contact of each electrode with cardiac wall tissue. EP signals from electrodes in contact with tissue have clinical relevance, while EP signals acquired in a blood pool are typically irrelevant.

Next, processor 38 determines from the multiple signals which electrodes 53 are to be active for ablation, at a preparation for ablation step 86.

Subsequently, processor 38 updates GUI 46 to partially blank EP signals from electrodes determined to be inactive for ablation, at a signal blanking step 88.

Note, as an example, in step 86, the processor may indicate, using contact-force sensing and/or impedance sensing, that one or more of electrodes 53 are not in firm enough contact with tissue (meaning they at least partially immersed in blood). The information on electrodes assists
physician 30 which electrodes to select, however the physician will not necessarily select all electrodes determined to active for ablation.

The example flow chart shown in Fig. 3 is chosen purely for the sake of conceptual clarity. In alternative embodiments, additional steps may take place, such as blinking electrodes/signals not in contact with tissue.

Although the embodiments described herein mainly address pulmonary vein isolation, the methods and systems described herein can also be used in other applications that require selecting electrodes, such as, for example, renal denervation, and generally, in ablating other organs in which electrode impedance or electrode temperature need to be highlighted.

It will thus be appreciated that the embodiments described above are cited by way of example, and that the present invention is not limited to what has been particularly shown and described hereinabove. Rather, the scope of the present invention is defined by the appended claims and thus includes both combinations and subcombinations of the various features described hereinabove, as well as variations and modifications thereof which would occur to persons skilled in the art upon reading the foregoing description and which are not disclosed in the prior art.

## Claims

1. A system for visualizing multiple signals sensed by multiple electrodes of a multi-electrode catheter, comprising:
a display (26); and
a processor (38), configured to:
receive (84) multiple signals sensed by multiple electrodes (53) of a multi-electrode catheter (40) in a heart of a patient (28);
determine (86) which of the electrodes are to be active in an ablation procedure; and
present (88) the multiple signals to a user (30), on the display, using a graphical user interface (GUI) (46) that (i) visualizes the signals from the electrodes determined to be active (55, 57) by a first graphical feature, and (ii) visualizes the signals from the electrodes determined not to be active (56) by a second graphical feature, different from the first graphical feature.

2. The system according to claim 1, wherein the processor is configured to visualize a signal using the first graphical feature by plotting the signal with a first line style, and using the second graphical feature by plotting the signal with a second line style, different from the first line style.

3. The system according to claim 1, wherein the processor is configured to visualize the signals from the electrodes determined not to be active by partially blanking the signals.

4. The system according to claim 1, wherein the processor is further configured to automatically update the visualization of the signals from the electrodes based on determining level of physical contact of electrode with tissue.

5. The system according to claim 1, wherein the processor is further configured to indicate to the user, using the GUI, which of the electrodes are determined to be active.

6. The system according to claim 5, wherein the processor is configured to indicate which of the electrodes are determined to be active on a graphical illustration of the catheter.

7. The system according to claim 1, wherein the signals are electrograms.

8. A computer software product, the product comprising a tangible non-transitory computer-readable medium in which program instructions are stored, which instructions, when executed by a processor (38), cause the processor to:
receive (84) multiple signals sensed by multiple electrodes (53) of a multi-electrode catheter (40) in a heart of a patient (28);
determine (86) which of the electrodes are to be active in an ablation procedure; and
present (88) the multiple signals to a user (30) on a display (26), using a graphical user interface (GUI) (46) that (i) visualizes the signals from the electrodes determined to be active (55, 57) by a first graphical feature, and (ii) visualizes the signals from the electrodes determined not to be active (56) by a second graphical feature, different from the first graphical feature.

## Patentansprüche

1. System zum Visualisieren mehrerer Signale, die durch mehrere Elektroden eines Mehrelektrodenkatheters erfasst werden, umfassend:
eine Anzeige (26); und
einen Prozessor (38), der konfiguriert ist zum:
Empfangen (84) mehrerer Signale, die durch mehrere Elektroden (53) eines Mehrelektrodenkatheters (40) im Herzen eines Patienten (28) erfasst werden;
Bestimmen (86), welche der Elektroden bei einem Ablationsverfahren aktiv sein sollen; und
Darstellen (88) der mehreren Signale für einen Benutzer (30) auf der Anzeige unter Verwendung einer grafischen Benutzerschnittstelle (GUI) (46), die (i) die Signale von den Elektroden, die durch ein erstes grafisches Merkmal als aktiv bestimmt werden (55, 57), visualisiert und (ii) die Signale von den Elektroden, die durch ein zweites grafisches Merkmal, das sich vom ersten grafischen Merkmal unterscheidet, als nicht aktiv bestimmt wurden (56), visualisiert.

2. System nach Anspruch 1, wobei der Prozessor konfiguriert ist, um ein Signal unter Verwendung des ersten grafischen Merkmals zu visualisieren, durch Aufzeichnen des Signals mit einem ersten Linienstil und unter Verwendung des zweiten grafischen Merkmals, durch Aufzeichnen des Signals mit einem zweiten Linienstil, der sich vom ersten Linienstil unterscheidet.

3. System nach Anspruch 1, wobei der Prozessor konfiguriert ist, um die Signale von den Elektroden, die als nicht aktiv bestimmt wurden, durch teilweises Ausblenden der Signale zu visualisieren.

4. System nach Anspruch 1, wobei der Prozessor ferner konfiguriert ist, um die Visualisierung der Signale von den Elektroden automatisch zu aktualisieren, basierend auf dem Bestimmungsgrad des physischen Kontakts der Elektrode mit dem Gewebe.

5. System nach Anspruch 1, wobei der Prozessor ferner konfiguriert ist, um dem Benutzer unter Verwendung der GUI anzugeben, welche der Elektroden als aktiv bestimmt sind.

6. System nach Anspruch 5, wobei der Prozessor konfiguriert ist, um auf einer grafischen Veranschaulichung des Katheters anzugeben, welche der Elektroden als aktiv bestimmt sind.

7. System nach Anspruch 1, wobei die Signale Elektrogramme sind.

8. Computersoftwareprodukt, das Produkt umfassend ein greifbares, nicht flüchtiges, computerlesbares Medium, auf dem Programmanweisungen gespeichert sind, wobei die Anweisungen, wenn sie durch einen Prozessor (38) ausgeführt werden, den Prozessor veranlassen zum:
Empfangen (84) mehrerer Signale, die durch mehrere Elektroden (53) eines Mehrelektrodenkatheters (40) im Herzen eines Patienten (28) erfasst werden;
Bestimmen (86), welche der Elektroden bei einem Ablationsverfahren aktiv sein sollen; und
Darstellen (88) der mehreren Signale für einen Benutzer (30) auf der Anzeige (26) unter Verwendung einer grafischen Benutzerschnittstelle (GUI) (46), die (i) die Signale von den Elektroden, die durch ein erstes grafisches Merkmal als aktiv bestimmt werden (55, 57), visualisiert und (ii) die Signale von den Elektroden, die durch ein zweites grafisches Merkmal, das sich vom ersten grafischen Merkmal unterscheidet, als nicht aktiv bestimmt wurden (56), visualisiert.

## Revendications

1. Système permettant la visualisation de signaux multiples détectés par des électrodes multiples d'un cathéter à électrodes multiples, comprenant :
une unité d'affichage (26) ; et
un processeur (38), configuré pour :
recevoir (84) des signaux multiples détectés par des électrodes multiples (53) d'un cathéter électrodes multiples (40) dans le coeur d'un patient (28) ;
déterminer (86) quelles électrodes doivent être actives dans une procédure d'ablation ; et
présenter (88) les signaux multiples à un utilisateur (30), sur l'unité d'affichage, à l'aide d'une interface utilisateur graphique (GUI) (46) qui (i) visualise les signaux provenant des électrodes déterminées comme étant actives (55, 57) par une première caractéristique graphique, et (ii) visualise les signaux provenant des électrodes déterminées comme n'étant pas actives (56) par une seconde caractéristique graphique, différente de la première caractéristique graphique.

2. Système selon la revendication 1, dans lequel le processeur est configuré pour visualiser un signal à l'aide de la première caractéristique graphique en traçant le signal avec un premier style de ligne, et à l'aide de la seconde caractéristique graphique en traçant le signal avec un second style de ligne, différent du premier style de ligne.

3. Système selon la revendication 1, dans lequel le processeur est configuré pour visualiser les signaux provenant des électrodes déterminées comme n'étant pas actives en supprimant partiellement les signaux.

4. Système selon la revendication 1, dans lequel le processeur est en outre configuré pour mettre automatiquement à jour la visualisation des signaux provenant des électrodes en fonction de la détermination du niveau de contact physique de l'électrode avec des tissus.

5. Système selon la revendication 1, dans lequel le processeur est en outre configuré pour indiquer à l'utilisateur, à l'aide de la GUI, lesquelles des électrodes sont déterminées comme étant actives.

6. Système selon la revendication 5, dans lequel le processeur est configuré pour indiquer quelles électrodes sont déterminées comme étant actives sur une illustration graphique du cathéter.

7. Système selon la revendication 1, dans lequel les signaux sont des électrogrammes.

8. Produit logiciel informatique, le produit comprenant un support tangible non transitoire lisible par ordinateur dans lequel sont stockées des instructions de programme, lesquelles instructions, lorsqu'elles sont exécutées par un processeur (38), amènent le processeur à :
recevoir (84) des signaux multiples détectés par des électrodes multiples (53) d'un cathéter électrodes multiples (40) dans le coeur d'un patient (28) ;
déterminer (86) quelles électrodes doivent être actives dans une procédure d'ablation ; et
présenter (88) les signaux multiples à un utilisateur (30) sur une unité d'affichage (26), à l'aide d'une interface utilisateur graphique (GUI) (46) qui (i) visualise les signaux provenant des électrodes déterminées comme étant actives (55, 57) par une première caractéristique graphique, et (ii) visualise les signaux provenant des électrodes déterminées comme n'étant pas actives (56) par une seconde caractéristique graphique, différente de la première caractéristique graphique.
